# DEMANDE DE BREVET EUROPEEN

(11) **EP 1 138 268 A1**
(43) Date de publication de la demande: **04.10.2001**
(21) Numéro de dépôt: 01400732.2
(22) Date de dépôt: 21.03.2001
(51) Int. Cl.: A61B 17/70

(54) **Dispositif de fixation d'une cale inter épineuse sur le sacrum**

(30) Priorité: 21.03.2000 FR 0003572; 12.02.2001 FR 0101877
(71) Demandeur: Cousin Biotech (S.A.S.), 59117 Wervicq Sud (FR); Taylor, Jean, 06400 Cannes (FR)
(72) Inventeur: Taylor, Jean, 06400 Cannes (FR); Deneuvillers, Guy, 62155 Merlimont (FR); Bardel, Frédéric, 59116 Houplines (FR)
(74) Mandataire: Viard, Jean

(57) **Abrégé**

Cale intervertébrale et dispositif de fixation constitué par une barre (12) de liaison, montée entre deux crochets (11), sur laquelle vient se clipser un évidement (22) d'une cale intervertébrale (10) dont le second évidement (21) est orthogonal à l'évidement (22).

## Description

La présente invention a pour objet un dispositif de fixation, notamment d'une cale inter épineuse, sur le sacrum et une cale adaptée à une telle fixation.

Dans l'anatomie du rachis, le sacrum est constitué par cinq vertèbres soudées entre elles. Cette formation a conduit à la quasi disparition des épineuses desdites vertèbres.

Il a déjà été proposé dans FR-B-1004625 une prothèse pour le blocage de l'articulation lombo-sacrée. Ce blocage permet d'éviter les douleurs lors de déplacements relatifs des vertèbres. Dans ce brevet, la prothèse prend appui sur les deux épineuses extrêmes et se compose de deux barres longitudinales, fixées dans les vertèbres, solidarisées entre elles au moyen d'une ou plusieurs traverses. Mais une telle prothèse immobilise complètement la région concernée.

Par ailleurs, notamment afin d'éviter des pincements des disques inter vertébraux, surtout vers l'arrière ce qui se traduit par des douleurs violentes, il est connu de disposer entre les apophyses épineuses des cales intervertébrales. Par ailleurs, notamment afin d'éviter des pincements des disques inter vertébraux, surtout vers l'arrière ce qui se traduit par des douleurs violentes, il est connu de disposer entre les apophyses épineuses des cales intervertébrales. Une telle cale est décrite notamment dans FR-A-2 775 183 au nom de TAYLOR. Elle présente deux oreilles définissant deux évidements dans le prolongement l'un de l'autre et dans chacun desquels viendra prendre appui une apophyse épineuse adjacente. Ces cales sont maintenues en position par contact des apophyses avec les bords de la cale et/ou par des ligaments entourant des épineuses entre lesquelles est insérée la cale. En bloquant une partie de la colonne vertébrale, elles produisent des transferts de charge au-dessus et en dessous des vertèbres entre les apophyses desquelles sont introduites des cales. Or, du fait de l'anatomie de la région sacrée la mise en place d'une cale inter épineuse sur le niveau L5,S1 n'est pas possible.

Les arthrodèses lombaires avec ostéosynthèse au moyen d'un matériel orthopédique implanté dans le rachis à l'aide de vis pédiculaires sont réputées induire, par un phénomène de rupture de contrainte, une sollicitation accrue du segment vertébral sous-jacent qui peut conduire à une dégénérescence discale. Les arthrodèses lombaires peuvent également accélérer une sténose suite à une hyper mobilité segmentaire à l'occasion d'une libération canalaire étendue et déstabilisante.

Un premier objet de l'invention est de pallier les inconvénients des dispositifs connus et de permettre l'insertion d'une cale intervertébrale en l'absence d'apophyse épineuse, notamment dans la région lombo-sacrée.

Selon l'invention, le dispositif de fixation d'une cale intervertébrale notamment dans la région sacrée du rachis est caractérisé en ce qu'il comprend des moyens pour assurer la fixation sur le sacrum d'un organe d'appui de la cale.

Selon une autre caractéristique de l'invention, le dispositif de fixation comprend deux vis pédiculaires et une barre de liaison avec les deux vis sur laquelle est monté, dans sa partie centrale, une plaque prenant appui, après sa pose, sur le sacrum.

La plaque centrale joue ainsi le rôle d'une épineuse pour maintenir la cale entre les vertèbres L5 et S1.

Selon une autre caractéristique de l'invention, une butée traversant la plaque autorise un contre appui sur le sacrum pour soutenir les charges appliquées par la cale inter épineuse.

Dans un autre mode de réalisation, la cale intervertébrale comprenant deux évidements est caractérisée en ce que les deux évidements sont sensiblement orthogonaux l'un à l'autre.

De préférence, un évidement présente une section en "V", le second étant de forme adaptée à la réception d'une barre ou tige notamment à section circulaire. La cale est réalisée en un matériau souple, par exemple en silicone recouvert de textile, dont la souplesse est réglée pour qu'elle constitue un soutien au niveau instrumenté. Elle s'adapte sur l'épineuse de la vertèbre supérieure ou inférieure au niveau arthrodésé et sur une barre ou tige de liaison fixée sur S1. La barre permet, en outre de limiter le déplacement de la cale.

L'invention concerne également un dispositif faisant appel à une telle cale et comprenant deux pinces et une barre ou tige transversale, la barre étant immobilisée par deux vis, la barre s'insérant, après fixation dans l'un des évidements de la cale. La tige joue ainsi le rôle d'une épineuse pour maintenir la cale entre les vertèbres L5 et S1.

D'autres caractéristiques et avantages de l'invention apparaîtront au cours de la description qui va suivre d'un mode particulier de réalisation, donné uniquement à titre d'exemple non limitatif, en regard des dessins qui représentent :
- La figure 1, une vue de côté de la plaque prothétique avec vis de fixation et cale intervertébrale;
- La figure 2, une vue de face de la barre de fixation;
- la figure 3, une vue par-dessus de la même barre;
- Les figures 4 et 5 des vues de détail de la plaque.
- La figure 6, une vue schématique de l'utilisation d'une cale selon un autre mode de réalisation de l'invention;
- La figure 7, une vue éclatée en perspective d'un dispositif selon l'invention dans le second mode de réalisation.

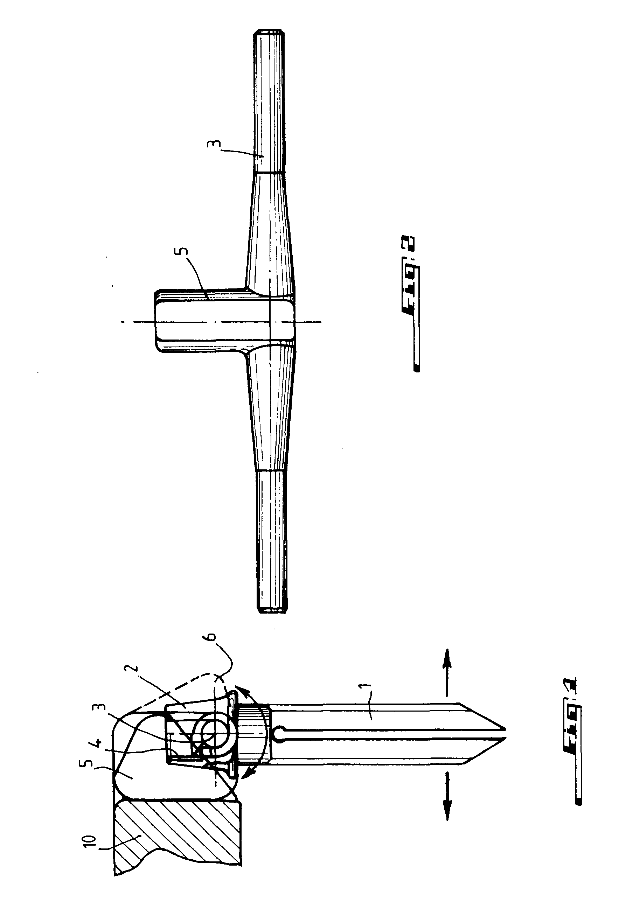

Sur l'ensemble des figures, les mêmes références désignent les mêmes éléments. Sur la figure 1, on voit que le dispositif de fixation se compose de deux vis pédiculaires 1 (dont une seule apparaît sur la figure) à l'intérieur des têtes 2 desquelles est glissée une barre 3. La tête tulipe de chacune des vis 1 est filetée pour recevoir un bouchon également fileté 4 qui immobilise la tige 3 en rotation. En effet, la barre 3 peut tourner avant fixation par les bouchons 4 pour permettre une adaptation de la fixation à la morphologie du patient (angle entre les vertèbres lombaires et le sacrum). De préférence, les vis 1 sont des vis à expansion jouant le rôle de cheville pour assurer une fixation solide dans l'os.

On a représenté partiellement sur la figure 1 en coupe une cale intervertébrale 10 qui prend appui d'une part sur L5 et, d'autre part, sur la plaque 5 elle-même fixée sur le sacrum. La cale souple peut légèrement pivoter par rapport aux vis 1. De préférence la plaque 5 présente un prolongement 6, vers l'arrière sur la figure, destiné à permettre son appui sur le sacrum (non représenté).

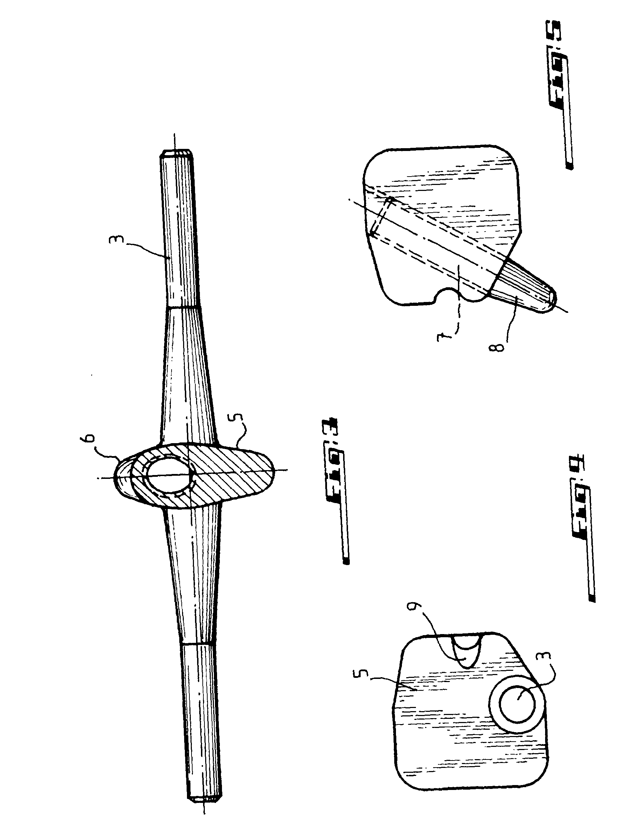

Sur la figure 2 on distingue la tige ou barre 3 dont les extrémités, éventuellement coupées à longueur, sont introduites, après vissage des vis 1, dans les têtes 2 dans lesquelles elles seront serrées. La plaque 5 présente une protubérance destinée à pallier l'absence d'épineuse à ce niveau. Elle est, bien entendu, orientée convenablement par rapport au sacrum sur lequel elle va reposer. La tige 3 et la plaque 5 sont réalisées en un métal biocompatible léger tel que du titane.

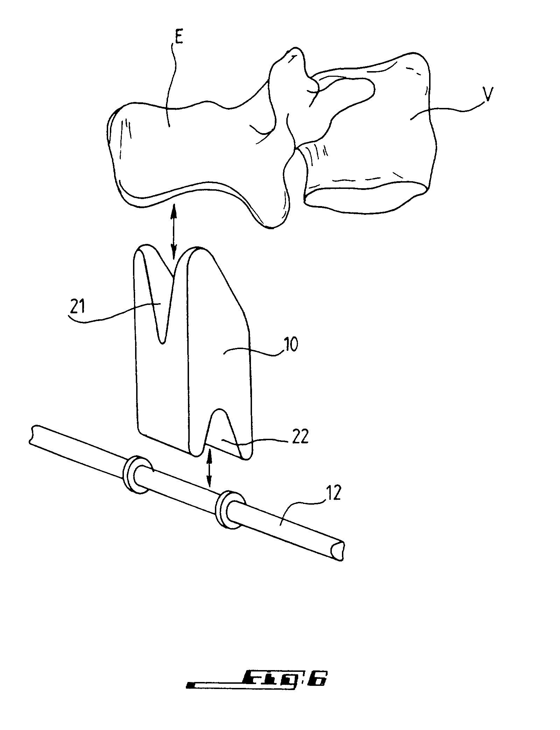

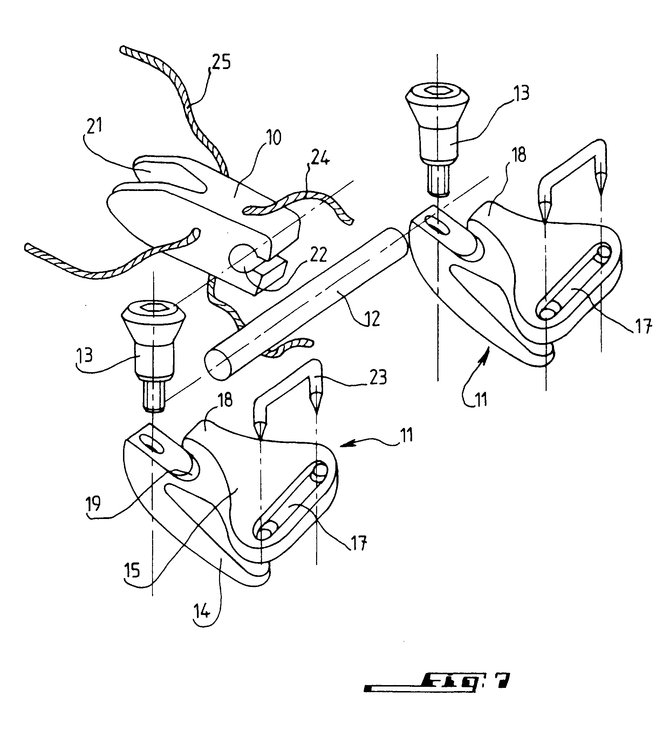

La plaque 5 représenté sur la figure 4 est inscriptible dans un rectangle. Elle présente dans sa partie arrière une échancrure 9 découpée pour permettre le maintien d'un ligament (non représenté) qui passe autour de l'apophyse de L5 et maintient la cale entre L5 et la plaque 5.

De préférence et comme représenté sur la figure 5, il est possible de faire passer un pied d'appui ou butée 8 faisant saillie à partir de la plaque. Cet appui est réglable pour adapter la prothèse à la morphologie du patient et est vissé dans la cavité 7 représentée sur la figure 5. Ce pied réglable peut être remplacé par une bille ou une pièce en « V » se stabilisant sur la partie supérieure du sacrum.

Le processus opératoire est le suivant :
- Dans un premier temps, le chirurgien perce dans le sacrum les trous de passage des vis 1 qu'il insère dans lesdits trous;
- Après quoi, il introduit la tige 3 dans les têtes 4 des vis pédiculaires 1 après, éventuellement, une mise à longueur et un cintrage de la tige;
- Il règle ensuite la position de la plaque 5 et assure son appui sur le sacrum par la butée réglable 8 et immobilise la tige 3 en rotation;
- Il pose la cale intervertébrale C et la fixe par un (ou deux) ligament.

Un autre mode de réalisation est représenté sur les figures 6 et 7.

Sur la figure 6, on distingue à la partie supérieure le corps vertébral V d'une vertèbre L5 se prolongeant, vers l'arrière, par une épineuse E. Après montage, l'épineuse E reposera dans un évidement 21 d'une cale 10. On a représenté sur la figure 6 une cale intervertébrale 10 qui prend appui d'une part sur l'épineuse de L5 par un évidement 21 et, d'autre part sur une tige 12, elle-même solidaire du sacrum, à l'intérieur d'un évidement 22.

La figure 7 représente le dispositif de montage d'une cale selon l'invention. On y retrouve la cale 10 et la barre 12 précédemment mentionnées. Il convient, dans un premier temps de fixer la barre 12 sur le sacrum.

Selon une caractéristique de l'invention, cette fixation est obtenue au moyen de deux pinces ou crochets 11 qui prennent appui sur l'arc postérieur de S1 (non représenté). Ces crochets sont fixés sur le sacrum, chacun par une agrafe 23, qui a pour but de positionner et de stabiliser les crochets. La branche inférieure 14 de chaque crochet est inclinée par rapport à sa partie supérieure 15 pour permettre au crochet de remonter lors de sa mise en place.

La partie supérieure arrière des crochets, en forme de "V" contient un logement 17 pour une agrafe 23. Dans la partie supérieure avant 18 de chaque crochet est prévue une encoche 19 de mise en place et de maintien de la barre 12. La barre peut ainsi être mise en place quelle que soit sa position dans l'espace. La fixation définitive de la barre est obtenue au moyen de deux vis 13 à tête fraisée.

Après la mise en place de la barre 12 sur les crochets 11, le dispositif est prêt à recevoir la cale inter épineuse 10 qui est, par exemple, en silicone recouverte d'un tissu, et comprend deux évidements 21,22 orthogonaux. Le premier évidement 21 reçoit la lame inférieure de l'épineuse L5 et le second la barre 12. Le second évidement 22 présente une forme appropriée pour recevoir la barre 12. Dans l'exemple représenté la barre étant à section circulaire, le second évidement 22 présente une ouverture adaptée pour que l'introduction de la barre provoque un clipsage par déformation de l'évidement 12.

Le positionnement de la cale peut être assuré par un ligament 25 qui entoure l'apophyse épineuse de la vertèbre correspondante. De même, la liaison tige cale peut être assurée par un ligament 24.

Le processus opératoire est le suivant :
- Dans un premier temps, le chirurgien pose les crochets 11 sur le sacrum, puis il pose les agrafes 23;
- Après quoi, il introduit la tige 12 dans les rainures 19 des crochets 11 après, éventuellement, une mise à longueur et un cintrage de la tige 12;
- Il immobilise la tige 12 en rotation par le vissage des vis 13;
- Il pose la cale intervertébrale 10 d'une part sur la tige 12 et, d'autre part sur l'épineuse supérieure et la fixe par un (ou deux) ligament 24,25.

L'invention autorise la mise en place d'une prothèse intervertébrale amortissante lors d'arthropathie facettaire, de déficit discal, dégénératif ou iatrogène, et d'ostéosynthèse transitionnelle. Elle peut être mise en oeuvre chaque fois que les apophyses sont absentes ou insuffisantes pour supporter une cale inter épineuse.

Il va de soi que de nombreuses variantes peuvent être apportées, notamment par substitution de moyens techniques équivalents, sans sortir pour cela du cadre de l'invention.

## Revendications

1. Dispositif de fixation d'une cale intervertébrale notamment dans la région sacrée du rachis **caractérisé en ce qu'**il comprend des moyens pour assurer la fixation sur le sacrum d'un organe d'appui de la cale (C,10).

2. Dispositif de fixation selon la revendication 1, comprenant deux vis pédiculaires (1) **caractérisé en ce qu'**il est constitué par une barre (3) de liaison avec les deux vis (1) sur laquelle est montée, dans sa partie centrale, une plaque (5) prenant appui, après sa pose, sur le sacrum et servant de support à une cale intervertébrale (10).

3. Dispositif de fixation selon la revendication 2, **caractérisé en ce qu'**une butée (8) traversant la plaque (5) autorise un contre appui sur le sacrum pour soutenir les charges appliquées par la cale (C).

4. Dispositif de fixation selon la revendication 2 ou 3, **caractérisé en ce que** la plaque (5) comporte dans sa partie arrière une échancrure (9) pour le passage d'un ligament.

5. Dispositif de fixation selon la revendication 2, 3 ou 4, **caractérisé en ce que** les extrémités de la barre (3) sont cylindriques et sont bloquées dans les têtes (2) des vis (1) par des bouchons (4).

6. Dispositif de fixation selon l'une des revendications précédentes, **caractérisé en ce que** les vis (1) sont expansibles latéralement.

7. Cale intervertébrale pour un dispositif de fixation selon la revendication 1, comprenant deux évidements longitudinaux (21,22) **caractérisée en ce que** les deux évidements sont sensiblement orthogonaux.

8. Dispositif de fixation selon la revendication 1 d'une cale selon la revendication 7, **caractérisé en ce qu'**il comprend deux crochets (11) s'accrochant sur l'arc postérieur du sacrum et une barre transversale (12), la barre (12) immobilisée par deux vis (13) s'insérant, après fixation, dans l'un des évidements (22) de la cale (10).

9. Dispositif de fixation selon la revendication 8, **caractérisé en ce que** chaque pince (11) comprend un logement (17) pour une agrafe (13).

10. Dispositif de fixation, selon la revendication 8 ou 9 **caractérisé en ce que** les extrémités de la tige (12) sont incluses dans deux rainures (19) des crochets (11), les vis (13) se vissant dans les crochets (11), bloquant la tige (12) à l'intérieur des rainures (19).

11. Dispositif de fixation selon l'une des revendications 6 à 9, **caractérisé en ce que** les crochets (11) présentent une branche (14) inclinée par rapport à leur surface supérieure (15).

12. Dispositif de fixation selon l'une des revendications précédentes, **caractérisé en ce que** la cale (10) est une cale en matériau souple et élastique.
